# EUROPEAN PATENT SPECIFICATION

(11) **EP 3 077 521 B1**
(45) Date of publication and mention of the grant of the patent: **08.08.2018**
(21) Application number: 14815085.7
(22) Date of filing: 08.12.2014
(51) Int. Cl.: C12N 15/90

(54) **NOVEL GENOME ALTERATION SYSTEM FOR MICROORGANISMS**
NEUARTIGES GENOMVERÄNDERUNGSSYSTEM FÜR MIKROORGANISMEN
NOUVEAU SYSTÈME DE MODIFICATION DU GÉNOME POUR MICRO-ORGANISMES

(30) Priority: 06.12.2013 NL 2011912
(43) Date of publication of application: 12.10.2016
(73) Proprietor: Heineken Supply Chain B.V., 1017 ZD Amsterdam (NL)
(72) Inventor: DARAN, Jean-Marc Georges, 2600 AA Delft (NL); GEERTMAN, Jan-Maarten Anton, 1017 ZD Amsterdam (NL); BOLAT, Irina, 2600 AA Delft (NL)
(74) Representative: V.O.
(86) International application number: PCT/NL2014/050839
(87) International publication number: WO 2015/084178

(56) References cited:
- QIAN WEIDONG ET AL: "Improved gene disruption method and Cre-loxP mutant system for multiple gene disruptions in Hansenula polymorpha", December 2009 (2009-12), JOURNAL OF MICROBIOLOGICAL METHODS, VOL. 79, NR. 3, PAGE(S) 253-259, XP002731664, ISSN: 0167-7012 figure 2 page 256, right-hand column, paragraph 2 - page 258, left-hand column, paragraph 2
- AKADA RINJI ET AL: "PCR-mediated seamless gene deletion and marker recycling in Saccharomyces cerevisiae", 15 April 2006 (2006-04-15), YEAST, JOHN WILEY & SONS LTD, GB, PAGE(S) 399 - 405, XP009138985, ISSN: 0749-503X [retrieved on 2006-04-05] cited in the application figure 1 page 401, left-hand column, paragraph 2 - right-hand column, paragraph 4

## Description

### Field

The invention relates to the fields of molecular biology and genetic engineering of microorganisms, especially of yeast.

### Introduction

Homologous recombination in microorganisms such as yeast is based on a double strand break repair mechanism, which joins the DNA fragments. When a double stranded DNA break is detected, an exonuclease degrades both 5' ends, after which strand invasion of homologous template takes place. The DNA synthesis mechanism repairs both strands and DNA ligation completes the process without any deletions [Storici et al., (2003). PNAS USA 100: 14994-9; Haber, (2000). Trends Genet 16: 259-264]. Although homologous recombination repair will occur with as little as 30 bp of homology, it is much more efficient with 200-400 bp [Sugawara et al., (2000). Mol Cell Biol 20: 5300-5309].

An alternative method to repair a double stranded break is based on non-homologous end joining, where the heterodimer of so called Ku proteins grasps the broken chromosome ends, which promotes the binding of additional proteins. These additional proteins process the DNA ends and ligate them, which generally creates a deletion of several nucleotides [Storici et al., (2003). PNAS USA 100: 14994-9].

The homologous recombination repair pathway was successfully used to construct a plasmid from two co-transformed DNA fragments, which contained homologous regions [Ma et al., (1987). Gene, 58: 201-16.26]. Microorganisms, and especially *S. cerevisiae* species, are tractable organisms for developing new techniques [Kumar and Snyder, (2001). Nat Rev Genet 2: 302-312], in which genetic alteration is either done with double stranded DNA or with single stranded DNA [Orr-Weaver et al., (1981). PNAS USA 78: 6354-6358; Moerschell et al., (1988). PNAS USA 85: 524-528]. *S. cerevisiae* can take up and assemble at least 38 overlapping single stranded oligonucleotides and a linear double-stranded vector in one transformation event with overlaps between oligonucleotides as few as 20 base pairs and with a length of 200 nucleotides [Gibson, (2009). Nucleic Acids Res 37: 6984-6990].

One of the most powerful tools in functional characterization of unknown gene products is the complete deletion of genes on a chromosome. Gene targeting has been established with PCR fragments flanked by homologous sequences as short as 35-40 bp that allow direct transformation due to the high efficiency of homologous recombination in *S. cerevisiae* [Baudin et al., (1993). Nucleic Acids Res 21: 3329-3330; Klinner and Schafer, (2004). FEMS Microbiol Rev 28: 201-223]. In the lager brewing *S. pastorianus* however, the efficiency of homologous recombination is low due to the complex genetics. Therefore, lager brewing strains necessitate longer homologous overlapping flanks (>400bp) in order to have an effective double strand breaks repair or insertion of a deletion cassette into genomic DNA.

To knock out multiple genes, marker recycling is also necessary. Previous systems for gene deletion and succeeding marker excision contained a marker either flanked by direct repeats of bacterial *hisG* sequence [Akada et al., (2002). Yeast 19: 393-402] or by two target sites of a site specific recombinase [McNabb et al., (1997). Biotechniques, 22: 1134-1139; Storici et al., (1999). Yeast 15: 271-283; Gueldener et al., (2002). Nucleic Acids Res 30: e23; Iwaki and Takegawa, (2004). Biosci Biotechnol Biochem 68: 545-550]. With this method, one copy of the repeats remains in the targeted chromosome. However, when multiple residual sequences are present in the genome, the percentage of correct integrations in successive counter-selectable cassette transformations is decreased dramatically [Davidson and Schiestl, (2000). Curr Genet 38: 188-190]. Multiple target sites can cause chromosomal rearrangements. For example, when four sets of *loxP* repeats were simultaneously located in the genome, chromosomal rearrangements occurred at a frequency of 50% by expression of the CRE recombinase. This means that successive targeting in the same microorganism requires more screening in each round to identify a correct knockout [Delneri et al., (2000). Gene 252: 127-135].

To overcome this problem, a system for seamless gene deletion in which a PCR-amplified cassette, containing a *URA3* marker attached to an duplicated 40 base pair sequence derived from the targeted locus, was used for *HIS3* disruption and marker recycling without any genomic scarring [Akada et al., (2006). Yeast 23: 399-405]. Colonies were counter selected using 5-fluoroorotic acid to identify colonies that had lost *URA3* by recombination between the duplicated 40 base pair sequences. This resulted in deletion of *HIS3* without residual extraneous sequences [Akada et al., (2006). Yeast 23: 399-405]. It was also shown that a long stretch of 966 base pairs was necessary for correct targeting of the gene. Replacement of this stretch by a short homologous sequence of 40 bp generated no transformants [Akada et al., (2006). Yeast 23: 399-405].

Targeting efficiency in part depends on the presence of a long homologous sequence in a targeting construct [Davidson and Schiestl, (2000). Curr Genet 38: 188-190]. However, homologous recombination in some microorganisms, such as for example most strains of the lager brewing yeast *Saccharomyces pastorianus,* is difficult to achieve, even in the presence of long homologous sequences in the targeting construct (Murakami et al., 2012. Yeast, 29: 155-165.

When homologous recombination is less efficient, the chance to get false positives increases. False positives usually are the results of random single cross over events.

The present invention overcomes the problem of efficient targeting by providing a set of targeting constructs, in which the correct expression of a selection marker depends on a recombination event between the targeting constructs. It was found that the occurrence of a recombination event between the targeting constructs is markedly enhanced after integration of the targeting constructs in the correct targeting locus. Therefore, the target system of the present invention, comprising a set of targeting constructs, greatly enhances the percentage of correctly integrated constructs in microorganisms that express the selection marker, compared to a one-vector targeting system. Splitting the marker in two limits the occurrence of false positives due to single cross over events. The split marker approach improves the ratio of true positives over false positives (Nielsen et al., 2006. Fungal Gen Biol 43: 54-64).

The invention provides a set of targeting constructs, comprising a first construct comprising a first region of homology with a target genome of a microorganism, a recognition site for an endonuclease, and a first part of a selection marker, and a second construct comprising a second part of the selection marker, a copy of the endonuclease recognition site and a second region of homology with the target genome of the microorganism, whereby the first and second regions of homology with the target genome each comprises at least 20 base pairs (bp), whereby a fragment of the first part of the selection marker overlaps with a fragment that is present in the second part of the selection marker, allowing recombination between the first and second part of the selection marker; said fragment preferably comprising between 50 and 600 bp, whereby a coding sequence that encodes the endonuclease and which is coupled to an inducible promoter is present on the first or second construct; and whereby a part of the first region of homology with the target genome on the first construct is duplicated between the copy of the endonuclease recognition site and the second region of homology with the target genome on the second construct; or a part of the second region of homology with the target genome on the second construct is duplicated between the first region of homology with the target genome and the endonuclease recognition site on the first construct, said duplicated region comprising between 20 and 200 bp.

Said first and second regions of homology with the target genome each comprises at least 20 base pairs (bp). There is in principle no upper limit for the length of said first and second regions of homology. However, for practical reasons such as ease and efficiency of generating the first and second constructs, said first and second regions of homology preferably comprise between 20 bp and 100 kb, more preferred between 40 bp and 10 kb, more preferred between 50 bp and 5 kb, more preferred between 100 bp and 1 kb.

Said duplicated region of homology with the target genome on the first and second targeting construct preferably is between 20 and 500 bp, preferably between 20 and 200 bp, preferably between 40 and 100 bp, preferably about 80 bp. Said duplicated region of homology with the target genome on the first and second targeting construct allows scarless removal of the marker from the target genome by homologous recombination.

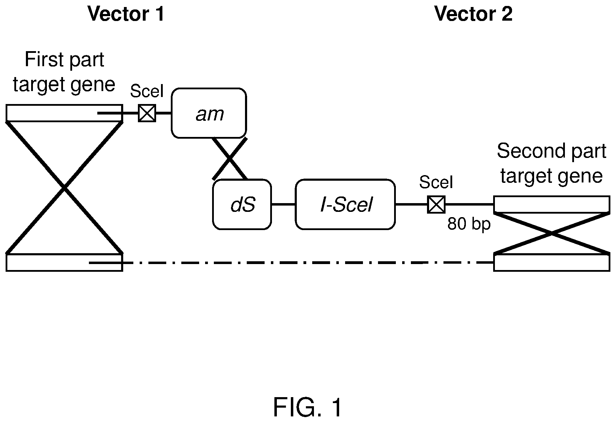

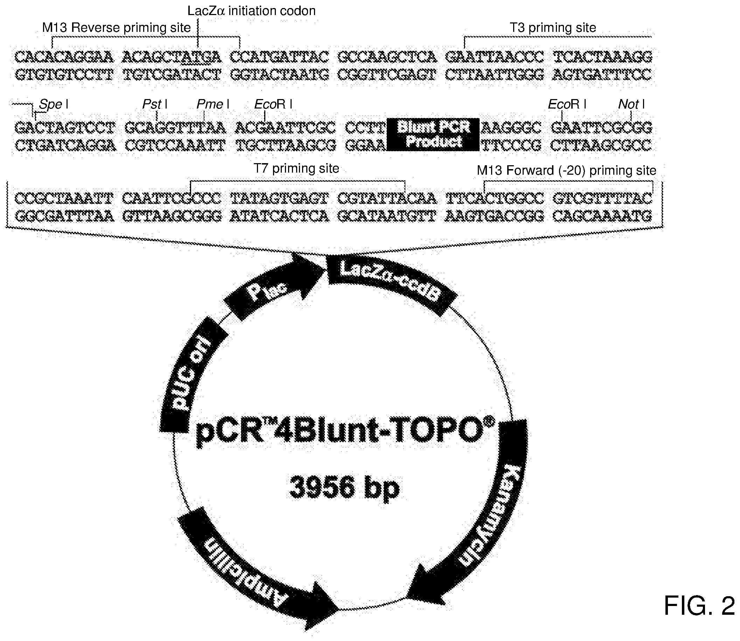

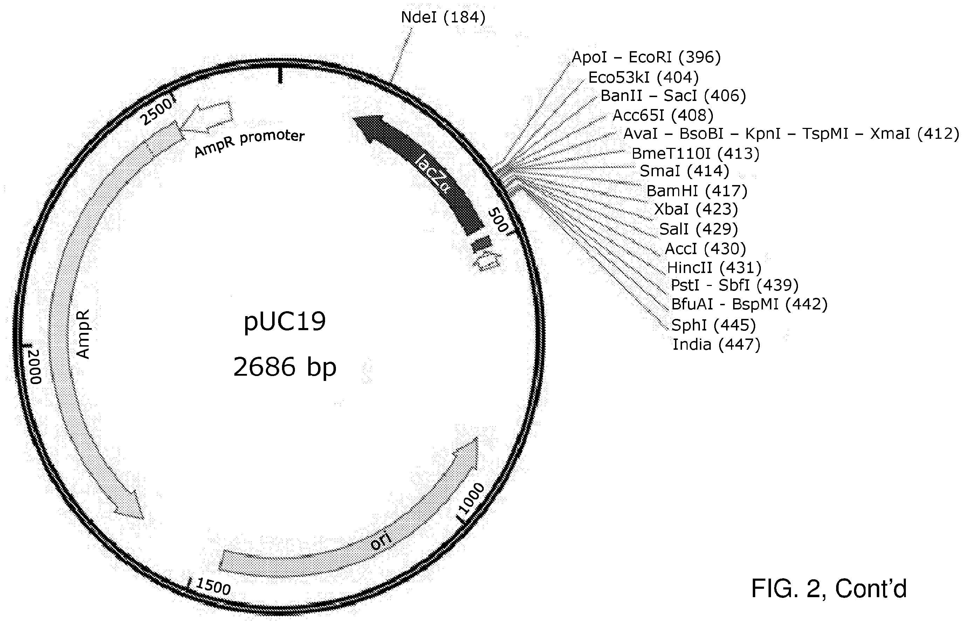

The first construct preferably comprises, in this order, a first region of homology with a target gene of a microorganism, a recognition site for an endonuclease, and a first part of a selection marker. The second construct preferably comprises, in this order, a second part of the selection marker, a coding sequence that encodes the endonuclease and which is coupled to an inducible promoter, a copy of the endonuclease recognition site, a copy of a part of the first region of homology with the target gene that is present on the first construct, and a second region of homology with the target gene of the microorganism. This configuration is depicted in Figure 1.

The term construct, as used herein, refers to an artificially constructed segment of nucleic acid. A preferred construct is a vector, preferably a vector that contains bacterial resistance genes for growth in bacteria. A most preferred construct is a plasmid, a linear or circular double-stranded DNA that is capable of replicating in bacteria independently of the chromosomal DNA.

The target gene can be any gene of a microorganism, preferably of a yeast, of which the genomic sequence is to be altered. The term gene, as is used herein, refers to a part of the genome of the microorganism that comprises intronic and exonic parts of a gene, the promoter region of said gene, and genomic sequences that mediate the expression of said gene, such as, for example enhancer sequences.

The skilled person will understand that the targeting constructs can preferably be used to alter a gene of a microorganism. Hence, the disclosure further provides a set of targeting constructs, comprising a first construct comprising a first region of homology with a target gene of a microorganism, a recognition site for an endonuclease, and a first part of a selection marker, and a second construct comprising a second part of the selection marker, a copy of the endonuclease recognition site and a second region of homology with the target gene of the microorganism, whereby a fragment of the first part of the selection marker overlaps with a fragment that is present in the second part of the selection marker, allowing recombination between the first and second part of the selection marker; whereby a coding sequence that encodes the endonuclease and which is coupled to an inducible promoter is present on the first or second construct; and whereby a part of the first region of homology with the target gene on the first construct is duplicated between the copy of the endonuclease recognition site and the second region of homology with the target gene on the second construct; or a part of the second region of homology with the target gene on the second construct is duplicated between first region of homology with the target gene and the endonuclease recognition site on the first construct. Said duplicated region of homology with the target gene on the first and second targeting construct preferably is between 20 and 200 bp, preferably between 40 and 100 bp, preferably about 80 bp.

The term alteration of the genomic sequence includes a replacement of one or more nucleotides, the insertion of one or more nucleotides, and/or the deletion of one or more nucleotides anywhere within a genome, preferably within a gene.

For example, if the first and second region of homology with a target gene comprise adjacent genomic sequences of the gene, a replacement of one or more nucleotides in the first region of homology, and/or in the second region of homology, will result in an alteration of the gene following homologous targeting with the set of targeting constructs according to the invention. Said replacement of one or more nucleotides preferably is in the region of homology with the target gene that is present on the first and on the second construct.

Said alteration of the genomic sequence preferably is a deletion of one or more nucleotides, preferably anywhere within the gene. For example, if the first and second region of homology with a target gene comprise genomic sequences of the gene that are separated on the genome of the organism, an alteration of the gene following homologous targeting with the set of targeting constructs according to the invention will result in a deletion of the region that was located between the first and second region of homology on the parental chromosome.

Said microorganism preferably is an aneuploid microorganism, preferably an aneuploid yeast. The term aneuploidy, as used herein, refers to presence of an abnormal number of chromosomes within a cell or an organism that differs from the normal number of chromosomes for that organism. An aneuploid microorganism may have one or more extra or missing chromosomes. The term aneuploid microorganism includes a polyploid microorganism. In fungi, aneuploidy is known to confer antifungal drug resistance and enables rapid adaptive evolution [Calo et al., (2013). PLoS Pathog 9(3): e1003181].

Said microorganism, preferably aneuploid microorganism, preferably is an *Ascomycota,* preferably a *Saccharomycotina,* preferably a *Saccharomyces sensu stricto (Saccharomyces paradoxus, S. mikatae, S. bayanus, S. eubayanus, S. kudriavzevii, S. paradoxus, S. arboricolus), Kazachstania, Naumovozyma, Nakaseomyces, Vanderwaltozyma, Zygosaccharomyces, Lachancea, Kluyveromyces, Eremothecium, Torulaspora, Ogataea, Debaryomyces, Clavispora, Candida, Komagataella, and*/*or Yarrowia* species. A preferred organism is the Lager brewing yeast *Saccharomyces pastorianus.*

*S. pastorianus* is supposed to be a hybrid of *S. cerevisiae* and *S. eubayanus.* The genome size of *S. pastorianus* is up to 60% larger than that of *S. cerevisiae,* and includes large parts of the two genomes. *S. cerevisiae* contains a haploid set of 16 chromosomes, ranging in size from 200 to 2,200 kb. The genome size of *S. pastorianus* is 24-50 Mb. Additionally reported aneuploid *Saccharomyces* species are *S. monacensis* and *S. uvarum.* An overview of polyploid fungi is provided by Albertin and Marullo, (2012). Proc R Soc B 279: 2497-2509.

Said selection marker is preferably an auxothrophic selection marker or a dominant selection marker, which are known to a skilled person. Preferred auxotrophic markers include *URA3, KlURA3; CaURA3; HIS3; his5; LEU2; KlLEU2; LYS2; TRP1; ADE1; ADE2;* and *MET15.* Preferred dominant markers include *KanMX; Sh ble; hph; CUP1; SFA1;* dehH1; *PDR3-9; AUR1-C; nat; pat; ARO4-OFP; SMR1; FZF1-4;* and *DsdA.* An overview of preferred markers that are routinely used in yeast organisms is provided in Table 1.

Said first construct preferably comprises a first part, preferably the first two-third or first half, of a region that encodes the selection marker. For example, *URA3,* also termed YEL021W, encodes the enzyme orotidine-5'-phosphate (OMP) decarboxylase which catalyzes the sixth enzymatic step in the de novo biosynthesis of pyrimidines, converting OMP into uridine monophosphate (UMP). The encoded protein has 267 amino acids, which is encoded by a nucleic acid sequence of 801 base pairs (bp). Said first construct preferably comprises between 200 and 600 bp of the coding region of *URA3,* more preferred between 300 and 500 bp. The second construct preferably comprises between 200 and 600 bp of the coding region of URA3, more preferred between 300 and 500 bp.

The region of overlap between the first and second part of the selection marker preferably is between about 20 bp and 800 bp, preferably between about 50 bp and about 600 bp, preferably about 200 bp.

A preferred selection marker is *URA3. URA3* encodes orotidine 5-phosphate decarboxylase (ODCase), which is an enzyme that catalyzes a reaction involved in the synthesis of pyrimidine ribonucleotides in yeast RNA. Loss of ODCase activity leads to a lack of cell growth unless uracil or uridine is added to the media. When a functional *URA3* gene is present, auxotrophic microorganisms can grow in the absence of uracil and/or uridine. In contrast, the addition of 5-fluoroorotic acid in the presence of a functional *URA3* gene results in the formation of a toxic compound, causing death of the microorganisms. Hence, *URA3* allows for both positive and negative selection.

A further preferred selection marker is provided by a nucleotide sequence encoding either agmatine ureohydrolase (agmatinase) (EC.3.5.3.11) or guanidino-acid hydrolase (guanidinobutyrase; EC.3.5.3.7). Microorganisms, preferably of the family *Saccharomytacea,* including *S. cerevisiae* strains, are not able to grow on guanidinobutyrate and/or agmatine as sole nitrogen source. Both guanidino-acid hydrolase and agmatinase catalyze the formation of urea, a nitrogen source commonly assimilated by microorganisms such as *S. cerevisiae.* Therefore, agmatinase and guanidinobutyrase present the essential characteristics of a potential dominant "gain of function" selectable marker in microorganisms such as *S. cerevisiae,* when grown on guanidinobutyrate and/or agmatine as sole nitrogen source. A preferred guanidinobutyrase gene encodes a protein comprising the amino acid sequence of GenBank XP_456325.1, or a enzymatically active part thereof. A preferred agmatine ureohydrolase gene encodes a protein comprising the amino acid sequence of GenBank AAC75974.1, or a enzymatically active part thereof.

Said selection marker is coupled to a promoter that directs expression of the selection marker in the microorganism, and a terminator that mediates efficient mRNA 3' end formation. Said promoter preferably is a yeast promoter, preferably a yeast promoter selected from a glycolytic gene *PGI1, PFK1, PFK2, FBA1, TPI1, TDH1, TDH3, PGK1, GPM1, ENOl, ENO2,* and from *ACT1, TEF1, AgTEF2, PMA1* promoter. Said promoter can also be employed to express a dominant selection marker. Terminators from a number of genes are known to the skilled person and have been employed, for example in expression vectors, including *CYC1, TRP1, ADH1, MFl, FLP* and *D* gene terminators (Romanos et al., 1992. Yeast 8: 423-488).

The first or second targeting construct comprises a coding sequence that encodes an endonuclease and which is coupled to an inducible promoter. The endonuclease preferably is a rare-cutting endonuclease such as, for example, PacI (target recognition sequence 5'-TTAATTAA); AscI (target recognition sequence 5'-GGCGCGCC), and AsiSI (target recognition sequence 5'-GCGATCGC). PacI, AscI and AsiSI are available from New England Biolabs. The endonuclease more preferably is a homing endonuclease. The term homing endonuclease refers to an endonucleases that is encoded either as freestanding genes within introns, as a fusion with a host protein, or as a self-splicing intein. A preferred list of homing endonucleases is provided in Table 2. Additional examples of homing nucleases are I-DirI, I-NjaI, I-NanI, I-NitI, F-TevI, F-TevII, F-CphI, PI-MgaI, I-CsmI, which are all known to the skilled person. Further examples of homing nucleases are provided in Benjamin K (patent application US2012/052582). A preferred homing nuclease is PI-PspI (New England Biolabs; recognition sequence 5'-TGGCAAACAGCTATTATGGGTATTATGGGT)) or PI-SceI (New England Biolabs; recognition sequence 5'-ATCTATGTCGGGTGCGGAGAAAGAGGTAAT). The coding sequences of most homing endonuclease are known. For example, the coding sequence of PI-SceI and of PI-PspI are available from public databases (GenBank accession number Z74233.1 and Genbank accession number U00707.1, respectively). The skilled person will understand that a sequence that differs from the publicly available sequence for a nuclease, may still encode the nuclease. For example, the term PI-PspI coding region includes a sequence that deviates from the publicly available sequence, for example by codon optimization, but which still expresses an active endonuclease that recognizes and digests the indicated target recognition sequence.

Said endonuclease is under control of an inducible promoter. The term inducible promoter, as is used herein, refers to a promoter of which the expression can be regulated. Inducible promoters are known to the skilled person. Examples of inducible promoters that have been employed in yeast are the *GAL1* promoter and the *GAL10* promoter, which both are inducible by galactose, the *SUC2* promoter, which is inducible by sucrose, the *MAL12* promoter, which is inducible by maltose; the *CUP1* promoter, which is inducible by copper, and the tetO7 and tetO2 promoters, which are both inducible by tetracycline [Gari et al., (1997). Yeast 13: 837-48; Yen et al., 2003). Yeast 20 1255-62]. A preferred inducible promoter is the *GAL1* promoter.

One recognition site comprising the target recognition sequence for the endonuclease, is located adjacent to (behind) the first region of homology with a target gene of a microorganism on the first construct. A copy of this recognition site is located adjacent to (in front of) the second region of homology with the target gene of the microorganism on the second construct. The skilled person will understand that when a part of the first region of homology with the target gene on the first construct is duplicated between the copy of the endonuclease recognition site and the second region of homology with the target gene on the second construct, said copy of the recognition site is located adjacent to (in front of) the duplication of the first region of homology with the target gene on the second construct. Alternatively, the recognition site is located adjacent to (behind) the duplicated part of the second region of homology with the target gene on the first construct when a part of the second region of homology with the target gene on the second construct is duplicated on the first construct. The selection marker, including promoter and terminator sequences, and the coding region of the endonuclease, including the inducible promoter, are between the recognition site on the first construct and the copy of this recognition site on the second construct.

The invention further provides a method for altering a genome, preferably a target gene, in a microorganism, comprising providing the set of targeting constructs according to the invention to said microorganism, and selecting a microorganism in which the genome has been altered. Said selection of a microorganism in which the genome has been altered is preferably accomplished by selection of a microorganism that functionally expresses a recombined selection marker.

As is indicated herein above, the occurrence of a recombination event between the targeting constructs is markedly enhanced after integration of the targeting constructs in the correct targeting locus. Hence, the presence of a functionally recombined selection marker is highly indicative for the presence of correctly integrated targeting constructs in the target genome and, therefore, of an altered genome in the microorganism.

As is indicated herein above, the terms altering, alteration and altered refer to a replacement of one or more nucleotides, the insertion of one or more nucleotides, and/or the deletion of one or more nucleotides anywhere within the target gene.

A replacement of one or more nucleotides can be accomplished by altering one or more nucleotides in the first region of homology and/or in the second region of homology. When the first region of homology and the second region of homology cover adjacent regions of the genome, preferably target gene, the integration of the targeting vectors will result in an alteration of the genome. When present, said replacement of one or more nucleotides is preferably accomplished by altering one or more nucleotides in the overlapping region of homology with the genome that is present on the first and on the second construct.

Said alteration of a genomic sequence preferably is a deletion of one or more nucleotides anywhere within a genome, preferably within a gene. For example, if the first and second region of homology with a target genome comprise genomic sequences that are separated on the genome of the organism, an alteration of the genome following homologous targeting with the set of targeting constructs according to the invention will result in a deletion of the region that was located between the first and second region of homology on the parental chromosome.

The invention further provides a method for producing a microorganism comprising an altered genome, preferably an altered gene, the method comprising providing the set of targeting constructs according to the invention to said microorganism, and selecting a microorganism in which the genome has been altered and that functionally expresses a recombined selection marker.

The method for producing a microorganism comprising an altered genome preferably comprises inducing the inducible promoter for expression of the endonuclease, thereby removing the selection marker and the coding region of the endonuclease, including the inducible promoter, from the target genome.

The disclosure further provides a microorganism, comprising a genomic alteration that is produced by the methods of the invention. When present, the duplicated regions of homology with the target genome on the first and second targeting construct ensure seamless marker removal from the target genome by homologous recombination. The resulting microorganism comprises only the alteration or alterations that were present on the first and/or second targeting construct, or that were induced by recombination of the targeting constructs into the targeting genome, such as an insertion into the targeting genome or a deletion from the targeting genome.

The invention further provides a microorganism, comprising a genomic alteration, preferably an alteration of a target gene, the alteration comprising an insertion of a functionally recombined selection marker and a coding sequence for an endonuclease that is coupled to an inducible promoter, whereby the target genome comprises one copy of a recognition sequence for the endonuclease on both sites of the insertion.

The invention further provides a method for producing a microorganism comprising an altered genome, the method comprising providing a microorganism comprising an alteration of the genome, preferably of a target gene, the alteration comprising an insertion of a functionally recombined selection marker and a coding sequence for an endonuclease that is coupled to an inducible promoter, whereby the target genome comprises one copy of a recognition sequence for the endonuclease on both sites of the insertion, and inducing the inducible promoter to remove the nucleic acid sequences in between the recognition sequences of the endonuclease. Again, when present, the duplicated regions of homology with the target gene on the first and second targeting constructs ensure seamless marker removal from the target genome by homologous recombination by providing the genomic DNA with a small homologous piece to re-connect the broken DNA strands efficiently. The resulting microorganism comprises only the alteration or alterations that were present on the first and/or second targeting construct, or that were induced by recombination of the targeting constructs into the targeting genome, such as an insertion into the targeting genome or a deletion from the genome, preferably an insertion into a targeted gene or a deletion of the targeted gene or a deletion from within the targeted gene.

**Table 1**

| **Auxotrophic markers** | | | |
|---|---|---|---|
| **Marker gene** | **Mode of action** | **Recyclable/Method** | **Reference** |
| *URA3* | Repairs uracil deficiency | Yes/negative selection with 5-FOA | Alani E, Cao L & Kleckner N (1987) A method for gene disruption that allows repeated use of URA3 selection in the construction of multiply disrupted yeast strains. Genetics 116: 541-545. |
| | | | Langlerouault F & Jacobs E (1995) A method for performing precise alterations in the yeast genome using a recyclable selectable marker. Nucleic Acids Res 23: 3079-3081. |
| *KlURA3* | Repairs uracil deficiency | Yes/negative selection with 5-FOA | Shuster JR, Moyer D & Irvine B (1987) Sequence of the Kluyveromyces lactis URA3 gene. Nucleic Acids Res 15: 8573-8573. |
| *CaURA3* | Repairs uracil deficiency | Yes/negative selection with 5-FOA | Losberger C & Ernst JF (1989) Sequence and transcript analysis of the C. albicans URA3 gene encoding Orotidine-5'-Phosphate Decarboxylase. Curr Genet 16: 153-157. |
| *HIS3* | Repairs histidine deficiency | No/-- | Wach A, Brachat A, Alberti-Segui C, Rebischung C & Philippsen P (1997) Heterologous HIS3 marker and GFP reporter modules for PCR-targeting in Saccharomyces cerevisiae. Yeast 13: 1065-1075. |
| *his5* | Repairs histidine deficiency | No/-- | Wach A, Brachat A, Alberti-Segui C, Rebischung C & Philippsen P (1997) Heterologous HIS3 marker and GFP reporter modules for PCR-targeting in Saccharomyces cerevisiae. Yeast 13: 1065-1075. |
| *LEU2* | Repairs leucine deficiency | No/-- | Brachmann CB, Davies A, Cost GJ, Caputo E, Li JC, Hieter P & Boeke JD (1998) Designer deletion strains derived from Saccharomyces cerevisiae S288C: a useful set of strains and plasmids for PCR-mediated gene disruption and other applications. Yeast 14: 115-132. |
| *KlLEU2* | Repairs leucine deficiency | No/-- | Zhang YP, Chen XJ, Li YY & Fukuhara H (1992) LEU2 gene homolog in Kluyveromyces lactis. Yeast 8: 801-804. |
| *LYS2* | Repairs lysine deficiency | Yes/negative selection with alpha-aminoadipate | Chattoo BB, Sherman F, Azubalis DA, Fjellstedt TA, Mehnert D & Ogur M (1979) Selection of lys2 mutants of the yeast Saccharomyces cerevisiae by the utilization of alpha-aminoadipate. Genetics 93: 51-65. |
| *TPP1* | Repairs tryptophan deficiency | No/-- | Brachmann CB, Davies A, Cost GJ, Caputo E, Li JC, Hieter P & Boeke JD (1998) Designer deletion strains derived from Saccharomyces cerevisiae S288C: a useful set of strains and plasmids for PCR-mediated gene disruption and other applications. Yeast 14: 115-132. |
| *ADE1* | Repairs adenine deficiency | No/-- | Nakayashiki T, Ebihara K, Bannai H & Nakamura Y (2001) Yeast [PSI+] "prions" that are crosstransmissible and susceptible beyond a species |
| *ADE2* | Repairs adenine deficiency | No/-- | Brachmann CB, Davies A, Cost GJ, Caputo E, Li JC, Hieter P & Boeke JD (1998) Designer deletion strains derived from Saccharomyces cerevisiae S288C: a useful set of strains and plasmids for PCR-mediated gene disruption and other applications. Yeast 14: 115-132. |
| *MET15* | Repairs methionine deficiency | Yes/negative selection with methyl-mercury | Singh A & Sherman F (1974) Association of methionine requirement with methyl mercury resistant mutants of yeast. Nature 247: 227-229. |

| **Dominant markers** | | | |
|---|---|---|---|
| **Marker gene** | **Mode of action** | **Recyclable/Method** | **Reference** |
| *KanMX* | Resistance to G418 | No/-- | Wach A, Brachat A, Pohlmann R & Philippsen P (1994) New heterologous modules for classical or PCR-based gene disruptions in Saccharomyces cerevisiae. Yeast 10: 1793-1808. |
| *ble* | Resistance to phleomycin | No/-- | Gatignol A, Baron M & Tiraby G (1987) Phleomycin resistance encoded by the ble gene from transposon Tn5 as a dominant selectable marker in Saccharomyces cerevisiae. Mol Gen Genet 207: 342-348. |
| *Sh ble* | Resistance to Zeocin | No/-- | Drocourt D, Calmels T, Reynes JP, Baron M & Tiraby G (1990) Cassettes of the Streptoalloteichus hindustanus ble gene for transformation of lower and higher eukaryotes to phleomycin resistance. Nucleic Acids Res 18: 4009-4009. |
| *hph* | Resistance to hygromycin | No/-- | Gritz L & Davies J (1983) Plasmid encoded Hygromycin-B resistance the sequence of Hygromycin-B phosphotransferase gene and its expression in Escherichia coli and Saccharomyces cerevisiae. Gene 25: 179-188. |
| *Cat* | Resistance to chloramphenicol | No/-- | Hadfield C, Cashmore AM & Meacock PA (1986) An efficient chloramphenicol resistance marker for Saccharomyces cerevisiae and Escherichia coli. Gene 45: 149-158. |
| *CUP1* | Resistance to Cu2+ | No/-- | Henderson RCA, Cox BS & Tubb R (1985) The transformation of brewing yeasts with a plasmid containing the gene for copper resistance. Curr Genet 9: 133-138. |
| *SFA1* | Resistance to formaldehyde | No/-- | Van den Berg MA & Steensma HY (1997) Expression cassettes for formaldehyde and fluoroacetate resistance, two dominant markers in Saccharomyces cerevisiae. Yeast 13: 551-559. |
| *dehH1* | Resistance to fluoroacetate | No/-- | Van den Berg MA & Steensma HY (1997) Expression cassettes for formaldehyde and fluoroacetate resistance, two dominant markers in Saccharomyces cerevisiae. Yeast 13: 551-559. |
| *PDR3-9* | Multi drug resistance | No/-- | Lackova D & Subik J (1999) Use of mutated PDR3 gene as a dominant selectable marker in transformation of prototrophic yeast strains. Folia Microbiol 44: 171-176. |
| *AUR1-C* | Resistance to aureobasidin | No/-- | Hashida-Okado T, Ogawa A, Kato I & Takesako K (1998) Transformation system for prototrophic industrial yeasts using the AUR1 gene as a dominant selection marker. FEBS Lett 425: 117-122. |
| *nat* | Resistance to nourseothricin | No/-- | Goldstein AL & McCusker JH (1999) Three new dominant drug resistance cassettes for gene disruption in Saccharomyces cerevisiae. Yeast 15: 1541-1553. |
| *CYH2* | Resistance to cycloheximide | No/-- | Delpozo L, Abarca D, Claros MG & Jimenez A (1991) Cycloheximide resistance as a yeast cloning marker. Curr Genet 19: 353-358. |
| *pat* | Resistance to bialaphos | No/-- | Goldstein AL & McCusker JH (1999) Three new dominant drug resistance cassettes for gene disruption in Saccharomyces cerevisiae. Yeast 15: 1541-1553. |
| *ARO4-OFP* | Resistance to o-Fluoro-DL-phenylalanine | No/-- | Cebollero E & Gonzalez R (2004) Comparison of two alternative dominant selectable markers for wine yeast transformation. Appl Environ Microb 70: 7018-7023. |
| SMR1 | Resistance to sulfometuron methyl | No/-- | Xie Q & Jimenez A (1996) Molecular cloning of a novel allele of SMR1 which determines sulfometuron methyl resistance in Saccharomyces cerevisiae. FEMS Microbiol Lett 137: 165-168. |
| *FZF1-4* | Increased tolerance to sulfite | No/-- | Cebollero E & Gonzalez R (2004) Comparison of two alternative dominant selectable markers for wine yeast transformation. Appl Environ Microb 70: 7018-7023. |
| *DsdA* | Resistance to D-Serine | No/-- | Vorachek-Warren MK & McCusker JH (2004) DsdA (D-serine deaminase): a new heterologous MX cassette for gene disruption and selection in Saccharomyces cerevisiae. Yeast 21: 163-171. |
| *amdS* | Enable growth on acetamide | Yes/ Fluoroacetamide | Selten G, Swinkels B & Van Gorcom R (2000) Selection marker gene free recombinant strains: A method for obtaining them and the use of these strains. Pat.no.US6051431. |
| | | | Swinkels B, Selten G, Bakhuis J, Bovenberg R & Vollebregt A (1997) The use of homologous *amdS* genes as selectable markers. Patent application WO97/06261. |

**Table 2**

| **Enzyme** | **Recognition sequence** | **Cut** | **SF** | **Source** | **D** | **SCL** |
|---|---|---|---|---|---|---|
| I-AniI | 5' TTGAGGAGGTTTCTCTGTAAAT AA 3' | 5'                    ---TTGAGGAGGTTTC TCTGTAAATAA--- 3' | HI | Aspergillus nidulans | E | mito |
| | 3' AACTCCTCCAAAGAGACATTTA TT 5' | 3'                    ---AACTCCTCC AAAGAGACATTTATT--- 5' | | | | |
| I-CeuI | 5' TAACTATAACGGTCCTAAGGTA GCGA 3' | 5'          ---TAACTATAACGGTCCTAA GGTAGCGA--- 3 | HI | Chlamydom onas eugametos | E | chloro |
| | 3' ATTGATATTGCCAGGATTCCAT CGCT 5' | 3'                ---ATTGATATTGCCAG GATTCCATCGCT--- 5' | | | | |
| I-ChuI | 5' GAAGGTTTGGCACCTCGATGTC GGCTCATC 3' | 5'            ---GAAGGTTTGGCACCTCG ATGTCGGCTCATC--- 3' | HI | Chlamydom onas humicola | E | chloro |
| | 3' CTTCCAAACCGTGGAGCTACAG | 3'                    ---CTTCCAAACCGTG GAGCTACAGCCGAGTAG--- 5' | | | | |
| | CCGAGTAG 5' | | | | | |
| I-CpaI | 5' CGATCCTAAGGTAGCGAAATTC A 3' | 5'          ---CGATCCTAAGGTAGCGAA ATTCA--- 3 | HI | Chlamydom onas pallidostig mata | E | chloro |
| | 3' GCTAGGATTCCATCGCTTTAAG T 5' | 3'                ---GCTAGGATTCCATC GCTTTAAGT--- 5' | | | | |
| I-CpaII | 5' CCCGGCTAACTCTGTGCCAG 3' | 5' ---CCCGGCTAACTC TGTGCCAG-- 3' | HI | Chlamydom onas pallidostig mata | E | chloro |
| | 3' GGGCCGATTGAGACACGGTC 5' | 5' ---GGGCCGAT TGAGACACGGTC--- 3' | | | | |
| I-CreI | 5' CTGGGTTCAAAACGTCGTGAG ACAGTTTGG 3' | 5' ---CTGGGTTCAAAACGTCGTGA GACAGTTTGG--- 3 | HI | *Chlamydo monas reinhardtii* | E | chloro |
| | 3' GACCCAAGTTTTGCAGCACTCT GTCAAACC 5' | 3'                ---GACCCAAGTTTTGCAG CACTCTGTCAAACC--- 5' | | | | |
| DmoI | 5' ATGCCTTGCCGGGTAAGTTCCG GCGCGCAT 3' | 5' --- ATGCCTTGCCGGGTAA GTTCCGG CGCGCAT--- 3' | | *Desulfuroco ccus mobilis* | A | Chrm |
| | 3' TACGGAACGGCCCATTCAAGG CCGCGCGTA 5' | 3'                                    --- TACGGAACGGCC    CATTCAAGGCC GCGCGTA--- 5' | | | | |
| H-DreI[ | 5' CAAAACGTCGTAAGTTCCGGC GCG 3' | 5'                                                    --- CAAAACGTCGTAA    GTTCCGGCGC G--- 3' | Hi | Escherichia coli | B | |
| | 3' GTTTTGCAGCATTCAAGGCCGC GC 5' | 3'                                                    --- GTTTTGCAG    CATTCAAGGCCGCG C--- 5' | | | | |
| I-HmuI | 5' AGTAATGAGCCTAACGCTCAGC AA 3' | :* 3'--- | HI II | *Bacillus* *subtilis*SPO 1 | B | phage |
| | | TCATTACTCGGATTGC GAGTCGTT --- 5' | | | | |
| | 3' TCATTACTCGGATTGCGAGTCG TT 5' | | | | | |
| I-HmuII | 5' AGTAATGAGCCTAACGCTCAAC AA 3' | 3'                                        --- TCATTACTCGGATTGCGAGTTGTTN NNNN--- 5' | HI II | *Bacillus subtilis* phage SP82 | B | phage |
| | 3'TCATTACTCGGATTGCGAGTT GTT 5' | | | | | |
| I-LlaI | 5' CACATCCATAACCATATCATTT TT 3' | 5' --- CACATCCATAA CCATATCATTTTT--- 3' | HI II | *Lactococcus lactis* | B | chrm |
| | 3' GTGTAGGTATTGGTATAGTAAA AA 5' | 3' --- GTGTAGGTATTGGTATAGTAA AA A--- 5' | | | | |
| I-MsoI | 5' CTGGGTTCAAAACGTCGTGAG ACAGTTTGG 3' | 5' --- CTGGGTTCAAAACGTCGTGA GAC AGTTTGG--- 3' | | *Monomasti* *x* sp. | E | |
| | 3' GACCCAAGTTTTGCAGCACTCT GTCAAACC 5' | 3' --- GACCCAAGTTTTGCAG CACTCTGT CAAACC--- 5' | | | | |
| PI-PfuI | 5' GAAGATGGGAGGAGGGACCGG ACTCAACTT 3' | 5' --- GAAGATGGGAGGAGGG ACCGGAC TCAACTT--- 3' | | Pyrococcus furiosus Vc1 | A | |
| | 3' CTTCTACCCTCCTCCCTGGCCT GAGTTGAA 5' | 3' --- CTTCTACCCTCC TCCCTGGCCTGA GTTGAA--- 5' | | | | |
| PI-PkoII | 5' CAGTACTACGGTTAC3' | 5' ---CAGTACTACG GTTAC--- 3' | | *Pyrococcus kodakaraen sis* KOD 1 | A | |
| | 3' GTCATGATGCCAATG5' | 3' ---GTCATG ATGCCAATG-5' | | | | |
| I-PorI | 5' GCGAGCCCGTAAGGGTGTGTA CGGG | 5' --- GCGAGCCCGTAAGGGT GTGTACG GG--- 3' | HI II | Pyrobaculu m organotrop hum | A | chrm |
| | 3' CGCTCGGGCATTCCCACACATG CCC | 3' --- CGCTCGGGCATT CCCACACATGC CC--- 5' | | | | |
| I-PpoI | 5' TAACTATGACTCTCTTAAGGTA GCCAAAT | 5' --- TAACTATGACTCTCTTAA GGTAGC CAAAT--- 3' | HI V | *Physarum polycephalu m* | E | nucle ar |
| | 3' ATTGATACTGAGAGAATTCCAT CGGTTTA | 3'                                    --- ATTGATACTGAGAG   AATTCCATCG GTTTA--- 5' | | | | |
| PI-PspI | 5' TGGCAAACAGCTATTATGGGTA TTATGGGT | 5' --- TGGCAAACAGCTATTAT GGGTATT ATGGGT--- 3' | HI | *Pyrococcus* sp. | A | chrm |
| | 3' ACCGTTTGTCGATAATACCCAT AATACCCA | 3' --- ACCGTTTGTCGAT AATACCCATAA TACCCA--- 5' | | | | |
| I-ScaI | 5' | 5' | HI | Saccharomyces capensis | E | mito |
| | TGTCACATTGAGGTGCACTAGT TATTAC | TGTCACATTGAGGTGCACT AGTTA TTAC--- 3' | | | | |
| | 3' ACAGTGTAACTCCACGTGATCA ATAATG | 3' --- ACAGTGTAACTCCAC GTGATCAAT AATG--- 5' | | | | |
| I-SceI | 5' AGTTACGCTAGGGATAACAGG GTAATATAG | 5' --- AGTTACGCTAGGGATAA CAGGGT AATATAG--- 3' | HI | *Saccharom yces cerevisiae* | E | mito |
| | 3' TCAATGCGATCCCTATTGTCCC ATTATATC | 3' --- TCAATGCGATCCC TATTGTCCCAT TATATC--- 5' | | | | |
| PI-SceI | 5' ATCTATGTCGGGTGCGGAGAA AGAGGTAATGAAATGGCA | 5' --- ATCTATGTCGGGTGC GGAGAAAG AGGTAATGAAATGGCA--- 3' | HI | *Saccharom yces cerevisiae* | E | |
| | 3' TAGATACAGCCCACGCCTCTTT CTCCATTACTTTACCGT | 3' --- TAGATACAGCC CACGCCTCTTTCT CCATTACTTTACCGT--- 5' | | | | |
| I-SceII | 5' TTTTGATTCTTTGGTCACCCTG AAGTATA | 5' --- TTTTGATTCTTTGGTCACCC TGAA GTATA--- 3' | HI | *Saccharom yces cerevisiae* | E | mito |
| | 3' AAAACTAAGAAACCAGTGGGA CTTCATAT | 3' --- AAAACTAAGAAACCAG TGGGACT TCATAT--- 5' | | | | |
| I-SecIII | 5' ATTGGAGGTTTTGGTAACTATT TATTACC | 5' --- ATTGGAGGTTTTGGTAAC TATTTA TTACC--- 3' | HI | *Saccharom yces cerevisiae* | E | mito |
| | 3' TAACCTCCAAAACCATTGATAA ATAATGG | 3' --- TAACCTCCAAAACC ATTGATAAAT AATGG--- 5' | | | | |
| I-SceIV | 5' TCTTTTCTCTTGATTAGCCCTA ATCTACG | 5' --- TCTTTTCTCTTGATTA GCCCTAAT CTACG--- 3' | HI | *Saccharomyces cerevisiae* | E | mito |
| | 3' AGAAAAGAGAACTAATCGGGA TTAGATGC | 3' --- AGAAAAGAGAAC TAATCGGGATT AGATGC--- 5' | | | | |
| I-SceV | 5' AATAATTTTCTTCTTAGTAATG CC | 5' --- AATAATTTTCT TCTTAGTAATGCC--- 3' | HI II | *Saccharomyces cerevisiae* | E | mito |
| | 3' TTATTAAAAGAAGAATCATTACGG | 3' --- TTATTAAAAGAAGAATCATTA CGG--- 5' | | | | |
| I-SceVI | 5' GTTATTTAATGTTTTAGTAGTT GG | 5' --- GTTATTTAATG TTTTAGTAGTTGG --- 3' | HI II | *Saccharom yces cerevisiae* | E | mito |
| | 3' CAATAAATTACAAAATCATCAA CC | 3' --- CAATAAATTACAAAATCATCA ACC --- | | | | |
| I-SceVII | 5' TGTCACATTGAGGTGCACTAGT TATTAC | **Unknown** | HI | *Saccharomyces cerevisiae* | E | mito |
| | 3' ACAGTGTAACTCCACGTGATCA ATAATG | | | | | |
| I-Ssp6803 I | 5' GTCGGGCTCATAACCCGAA | 5' ---GTCGGGCT CATAACCCGAA--- 3' | | *Synechocystis* sp. PCC 6803 | B | |
| | 3' CAGCCCGAGTATTGGGCTT | 3' ---CAGCCCGAGTA TTGGGCTT-5' | | | | |
| I-TevI | 5' AGTGGTATCAACGCTCAGTAGA TG | 5' --- AGTGGTATCAAC GCTCAGTAGAT G--- 3' --- TCACCATAGT TGCGAGTCATCTAC-5' | HI I | *Escherichia coli* phage T4 | B | phage |
| | 3'                    TCACCATAGT TGCGAGTCATCTAC | | | | | |
| I-TevII | 5' GCTTATGAGTATGAAGTGAACA CGTTATTC | 5' --- GCTTATGAGTATGAAGTGAACACGT TATTC--- 3' | HI I | *Escherichia coli* phase T4 | B | phase |
| | 3' CGAATACTCATACTTCACTTGT GCAATAAG | 3'                                    --- CGAATACTCATACTTCACTTGTG   C AATAAG--- 5' | | | | |
| I-TevIII | 5' TATGTATCTTTTGCGTGTACCT TTAACTTC | 5' --- T ATGTATCTTTTGCGTGTACCTTT AACTTC--- 3' | HI II | *Escherichia coli* phase RB3 | B | phase |
| | 3' ATACATAGAAAACGCACATGGA AATTGAAG | 3' --- AT ACATAGAAAACGCACATGGAA ATTGAAG--- 5' | | | | |
| PI-TliI | 5' TAYGCNGAYACNGACGGYTTY T | 5' --- TAYGCNGAYACNGACGG YTTYT-3' | HI | *Thermococcus litoralis* | A | chrm |
| | 3' ATRCGNCTRTGNCTGCCTAARA | 3' --- ATRCGNCTRTGNC TGCCTAARA-5' | | | | |
| PI-TliII | 5' AAATTGCTTGCAAACAGCTATT ACGGCTAT | **Unknown** ** | HI | *Thermococc us litoralis* | A | chrm |
| | 3' TTTAACGAACGTTTGTCGATAA TGCCGATA | | | | | |
| I-Tsp061I | 5' CTTCAGTATGCCCCGAAAC | 5' ---CTTCAGTAT GCCCCGAAAC--- 3' | | *Thermoproteus* sp. IC-061 | A | |
| | 3' GAAGTCATACGGGGCTTTG | 3' ---GAAGT CATACGGGGCTTTG-5' | | | | |
| I-Vdi141I | 5' CCTGACTCTCTTAAGGTAGCCA AA | 5' --- CCTGACTCTCTTAA GGTAGCCAAA --- 3' | | Vulcanisaeta distributa IC-141 | A | |
| | 3' GGACTGAGAGAATTCCATCGG TTT | 3' --- GGACTGAG AGAATTCCATCGGTT T--- 5' | | | | |

Table 2. overview of homing endonucleases and their target sequences.
Abbreviations: **SF** (Structural family): HI: LAGLIDADG family; HII: GIY-YIG family; HIII: H-N-H family; HIV: His-Cys box family.
**D:** Biological domain of the source: A: archaea; B: bacteria; E: eukarya. **SCL:** Subcelullar location: chloro: chloroplast; chrm: chromosomal; mito: mitochondrial; nuclear: extrachromosomal nuclear; phage: bacteriophage.

### Figure legends

Figure 1
   Vector 1 and 2 with all essential parts for the standard deletion cassette. The 400 base overlap in the selection marker amDs (indicated by a cross) is designed to recombine due to the homology.
Figure 2
   Non-directional TOPO Blunt cloning vector and pUC19 used for plasmid construction with vector 1 and 2.
Figure 3
   3A: Targeted gene deletion using the amdS-I-SceI cassette with 500 bp homologous flanks for knocking out ScARO10.
   3B: Seamless marker removal using the GAL1p of I-SceI to get the active endonuclease I-SceI protein, which restricts the genomic DNA at the cassette introduced SceI recognition sites.
Figure 4
   Vector 1 and 2 comprising overlap fragments of the selection marker *KlGBU1,* encoding a guanidinobutyrase.

### Examples

### Example 1

### Materials and methods

### Strains and cultivation conditions

All *Saccharomyces strains* used in this study are listed in Table I. For growth in liquid media, shake flasks were put in the incubator shaker at 200 rpm and 30°C. For growth on plates, all strains except CBS1483 were grown at 30°C. CBS1483 was grown at 20°C. The strains have been grown in different media: Under nonselective conditions, yeast was grown in complex medium Yeast Peptone Dextrose (YPD) containing 10 g/L yeast extract, 20 g/L peptone, 22 g/L glucose 1 hydrate, pH 6), Synthetic Media (SM) containing 5.0 g/L (NH₄)₂SO₄, 3.0 g/L KH₂PO₄, 0.5 g/L MgSO₄.7H₂O, 1 mL/L trace elements solution and 1 mL/L of a vitamin solution (Verduyn et al., 1992) were used [Verduyn et al., (1990). J General Microbiology 136: 395-403].

When amdSYM (*AgTEF2-amdS-AgTEF2ter*) was used as marker, (NH₄)₂SO₄ was replaced by 0.6 g/L acetamide as nitrogen source and 6.6 g/L_1K₂SO₄ to compensate for sulfate supply (SM-Ac). Recycled markerless cells were selected on SM containing 2.3 g/L fluoroacetamide (SM-Fac). SM, SM-Ac, and SM-Fac were supplemented with 20 mg/L adenine and 15 mg/L L-canavanine sulfate when required. In all experiments, 20 g/L of glucose was used as carbon source. The pH in all the media was adjusted to 6.0 with KOH. Solid media were prepared by adding 2% agar to the media described above.

For induction of the *GAL1p,* the carbon source glucose was replaced by the alternative carbon source galactose with a concentration of 20 g/L. When liquid galactose medium was used, growth was stimulated with addition of 0,0125% glucose. For stocking purposes, yeasts that had correctly assembled the transformation fragments into the genomic DNA, were grown in liquid YPD or MM culture. Yeasts with a correctly assembled plasmid, were grown under selective circumstances in a MM culture. After sufficient OD660 was reached, glycerol was added to obtain a concentration of 30% and the cells were stocked in a -80°C.

### DNA techniques

### Polymerized chain reaction (PCR)

Two different PCR methods were performed. For sequencing and cloning purposes, Phusion High Fidelity DNA polymerase (Finnzymes, Vantaa, Finland) was applied in order to have a more accurate reading, because it has 3' to 5' exonuclease proofreading activity. For selection procedure (yeast colony PCR) DreamTaq PCR master mix (Fermentas GmbH, St. Leon-Rot, Germany) was used, which lacks the 3' to 5' exonuclease proofreading activity.

**Table I. Saccharomyces species used in Example 1.**

| ***Strain*** | ***Genotype or description*** | ***Source or reference*** |
|---|---|---|
| CEN.PK113-7D | *MATa MAL2-8c SUC2* | van Dijken et al (2000), Entian & Kotter (2007) |
| CEN.PK122 | Diploid | van Dijken et al (2000), Entian & Kotter (2007) |
| PRG410 | Ale brewing strain *Saccharomyces cerevisiae* | Gift from Dr. JM Geertman (Heineken Supply Chain, Zoeterwoude, the Netherlands) |
| CMBS33 | Lager brewing strain *Saccharomyces pastorianus* | KU Leuvena |
| CBS1483 | Lager brewing strain *Saccharomyces pastorianus* | The Centraalbureau voor Schimmelcultures (CBS) Fungal Biodiversity Centre, (http://www.cbs.knaw.nl/index.php) |
| CBS12357 | *Saccharomyces eubayanus* sp.nov | The Centraalbureau voor Schimmelcultures (CBS) Fungal Biodiversity Centre, (http://www.cbs.knaw.nl/index.php) Libkind *et al.* (2011) |
| IMK486 | *MATa MAL2-8c SUC2 ar010*Δ-*AgTEF2pr-amdSAgTEF2ter*-GAL1p-*ISCEI-CYC 1ter* | This study |
| IMK487 | *MATa MAL2-8c SUC2 ar010*Δ-*AgTEF2pr-amdSAgTEF2ter-*GAL1p-*ISCEI-CYC 1ter* | This study |
| IMK488 | PRG410 *aro10*Δ-*AgTEF2pr-amdSAgTEF2ter*-GAL 1p*-ISCEI-CYC1ter* | This study |
| IMK489 | CMBS33 *aro10*Δ-*AgTEF2pr-amdSAgTEF2ter-*GAL 1p*-ISCEI-CYC1ter* | This study |
| IMK490 | CBS1483 *aro10*Δ-*AgTEF2pr-amdSAgTEF2ter-*GAL 1p*-ISCEI-CYC1ter* | This study |

| | | |
|---|---|---|
| ^{a} K.U. Leuven, Centre for Malting and Brewing Collection, Centre for Malting and Brewing, Louvain, Belgium. | | |

References: van Dijken et al., (2000). Enzyme Microb Technol 26: 706-714; Entian and Kotter, (2007). 25 yeast genetic strain and plasmid collections. Academic Press, Amsterdam, the Netherlands. In: Methods in Microbiology (Stansfield I & Stark J, eds) 36: 629-666 The Centraalbureau voor Schimmelcultures (CBS) Fungal Biodiversity Centre, (http://www.cbs.knaw.nl/index.php), Libkind et al., (2011). PNAS USA 108: 14539-14544.

In order to generate proper DNA template when performing yeast colony PCR, a small fraction of single colony was resuspended in 15 □L of 0.02 N NaOH and the yeast colony suspension was boiled for 10 min at 100°C. After what 2 µL of this cell suspension was used as template for the PCR that was performed using DreamTaq PCR master mix (Fermentas GmbH, St. Leon-Rot, Germany) following the manufacturer recommendations.

High-fidelity colony PCR was used to confirm insertion of the gene deletion cassette into the genomic DNA. Cells from a liquid yeast culture (200 pL) were spinned down, re-suspended in 100 pL of 0.2M LiAc with 1% of SDS solution and incubated for 5 minutes at 70°C. Addition of 96% ethanol and vortexing roughly, was followed by spinning down for 3 minutes at 15,000g. The pellet was washed with 70% ethanol and spinned down again. Ethanol was removed and the pellet was dried at a maximum temperature of 30°C, after which 100 pL of TE buffer was added to dissolve the pellet. Cell debris was spinned down for 30 seconds at 15,000g and 5 pL of the transferred supernatant was used for High-Fidelity colony PCR.

All primers used in PCR are described in Table II .

**Table II: Sequences of the primers used**

| **Cassette integration primers** | |
|---|---|
| KanA r | CGCACGTCAAGACTGTCAAG |
| KanB f | TCGTATGTGAATGCTGGTCG |
| KanA f | CTTGACAGTCTTGACGTGCG |
| KanB r | CGACCAGCATTCACATACGA |
| Fw 5'ScARO10/amdS check | ACAAGTTGACGCGACTTCTGTAAAG |
| Rv 3'ScARO10/amdS check | CAACTGGACAAAGAACTCTGTGGTAG |
| FK072 | CTCGAGTCATGTAATTAGTTATG |
| ScARO10-Fw inside | GGTGTGGCCAAGTCCATAG |
| ScARO10-Rv inside | CCTGTTTCACAAACGACAACATC |

| **Primer name** | **Sequence 5' to 3'** |
|---|---|
| **TOPO Blunt Primers** | |
| M13f | GTAAAACGACGGCCAG |
| M13r | CAGGAAACAGCTATGA |
| Fw V1 NdeI | GCGCATATGCGTCAGCAGAACCGTCAGCA |
| Rv V1 SceI SacI | GCGGAGCTCATTACCCTGTTATCCCTAAGGTCTAGAGATCTGTTTAGCTTGCC |
| Fw V2 NdeI | GCGCATATGAGATTGCCATACGCTAAGATGG |
| Rv V2 overlap ISceI | GCGACGCACGGAGGAGAGTCTTCCGTCGGAGGGCTGTCGCCCGCTCGGCGG |
| | CTTCTAATCCGTATTAAGGGTTCTCGAGAGCTCC |
| Fw V2 ISceI | GCGACGGATTAGAAGCCGCCGAG |
| Rv V2 SceI EcoRI | GCGGAATTCATTACCCTGTTATCCCTACAAATTAAAGCCTTCGAGCGTCC |
| Fw 5' ScARO10 KpnI | GCGGGTACCATGGCACCTGTTACAATTGAAAAGTTCG |
| Fw 5'ScARO10 NotI | GCGGCGGCCGCATGGCACCTGTTACAATTGAAAAGTTCG |
| Rv 5' ScARO10 SacI | GCGGAGCTCCTACCGAGCAAGCGACTCTATCTT |
| Fw 3' ScARO10 BamHT 80bp | GCGGGATCCAGCTACATGATTCAAATTTAAAGGGCCAAATCATAAAGTATAT CATGATATGGTAAAAGATAGAGTCGCTTGCTCGGTACATTGGCATGGCCCTT CCTG |
| Rv 3' ScARO10 PstI | GCGCTGCAGCTATTTTTTATTTCTTTTAAGTGCCGCTGC |

### Restriction

Restriction enzymes used in this project were supplied by Fermentas. The digestion mix was either prepared in a total volume of 20 pL or 30 pL depending on the final amount desired. A general mix contained 1 pL of each restriction enzyme (1 FastDigest unit/µL), 2 or 3 pL of 10x FastDigest buffer and nuclease free demineralized water. Approximately 50-200ng DNA was used in a total mix of 20µL and 100ng - 2pg in a total mix of 30pL. The digestion mix was incubated at a temperature of 37°C.

### Gel electrophoresis

To analyze different DNA techniques, nucleic acid molecules were loaded on 1% agarose gel (stained with 0.001% SybrSafe in advance) in 1x TAE buffer (40 mM Tris acetate and 1 mM EDTA). All DNA molecules were separated by applying an electric field of 100V for 30 min. Gel pictures were taken by exposing to UV light.

### Gel recovery

To purify DNA from gel, a gel extraction Kit (Sigma-Aldrich, Zwijndrecht, The Netherlands) was used. DNA fragment of interest was excised from the agarose gel with a blade. The DNA was extracted following the manufacturer recommendations.

### Ligation

Sticky or blunt ends of DNA fragments were joined by using T4 DNA Ligase (Life Technologies Europe BV, Bleiswijk, The Netherlands). Ligation mixture was prepared according to the standard manufacturers protocol and incubated at 16°C for 20 hours. The plasmids generated after ligation were ready to transform into *E. coli.*

### E. coli transformation

A small volume of the plasmids (5 µL) was added to 50 µL One Shot TOP10 chemically competent cells (Life Technologies) and the protocol for chemical transformation of *E. coli* from the supplier was followed. Transformed cells were incubated at 37°C for one hour with shaking, allowing the cells to recover and build up the resistance to the used antibiotic. The cell suspension was plated on pre-warmed selective plates and incubated overnight at 37°C. Cells with plasmids resulting from non-directional Blunt End TOPO cloning (Life Technologies) were plated on LB-agar plates (10 g tryptone, 5 g yeast extract, 10 g NaCl, 15 g/L agar) containing 50 µg/mL kanamycin (125 µL) or 100 µg/mL ampicillin (125 µL). Cells with plasmids, that resulted from cutting and ligation in pUC19, were streaked on LB with 100 µg/mL ampicillin (250 µL). After overnight incubation, single colonies were re-streaked on fresh pre-warmed selective plates to grow overnight again. After re-streaking, selection of single E. coli colonies was possible without background. Single colonies were inoculated into 5 mL LB medium with the appropriate antibiotic for overnight growth. Before isolation of the plasmids, 200 µL of glycerol solution was added to 800 µL of the culture, after which the sample was stored in -80°C freezer.

### Plasmid isolation

To isolate plasmids, an *E. coli* culture with the required plasmid was grown overnight in LB medium. After pelleting a sample of 1-5 mL, the GenElute Plasmid Miniprep kit (Sigma-Aldrich) protocol was followed. At the last step 50 µL instead of 100 µL nuclease free demineralised water was used to elute the purified plasmid from the column.

### Yeast transformation

For yeast transformation, the optical density (OD) was determined by the absorbance measured with a spectrophotometer at wavelength of 660 nm. With an OD660 between 0.6-0.8 (2×107 cells/mL for 10 transformations) or corrected for the amount of cells required for an individual transformation, the transformation was performed according to the lithium acetate single-stranded carrier DNA-polyethylene glycol method [Gietz and Woods, (2002). Methods in Enzymology 350: 87-96]. Yeast cells were streaked on corresponding selective plates.

### Plasmids construction

The central part of the complete gene deletion cassette, which will be called amdS-ISceI, consisted of the codon-optimized *amdS* gene and the *I-SCEI* under the galactose 1 promoter (*GAL1p*) flanked by two SceI recognition sites. The deletion cassette amdS-ISceI was separated into two fragments, which contained a 400 base pair overlap within the *amdS* gene to make a homologous recombination step an essential part in the in vivo assembly of the cassette. Primer sequences are provided in Table II.

The first fragment, named vector 1, was constructed with the reverse primer Rv V1 SceI SacI, which binds to the start of the amdSYM cassette at the AgTEF2 promoter and which included an SceI recognition site and the SacI restriction site, and with the forward primer Fw V1 NdeI, which bound to the end of the intended 400 base overlap within *amdS* and included a NdeI restriction site at its 5' end (Figure 1). pUGamdS was used as template for vector 1 construction.

For plasmid construction, the PCR fragment vector 1 described above was cloned in pCR4Blunt-TOPO yielding the plasmid pUD266.

The last part of *amdS* was amplified from pUGamdS [Solis-Escalante, D. et al. (2013). FEMS Yeast Research, 13:126-39] using the forward primer Fw V2 NdeI, that bound at the beginning of the intended 400 base pair overlap and included a NdeI restriction site to the PCR-product, and the reverse primer Rv V2-overlap-ISceI, to create an 60 base pair overlap to the *GAL1* promoter that controlled the expression of *I-SCEI.* This fragment was named vector 2A. The GAL1p*-I-SCEI* expression cassette was amplified from pUDC073 (Kuijpers et al., 2013. FEMS Yeast Research. 13:769-81) with the forward primer Fw V2 ISceI and the reverse primer Rv V2 SceI EcoRI, which included a SceI site at the other side of the cassette and an EcoRI site. This fragment was named vector 2B. The PCR-products, vector 2A and 2B were fused via PCR with the Fw V2 NdeI and Rv V2 SceI EcoRI. The PCR was performed by mixing equal molar amounts of both fragments resulting in a fusion fragment, named vector 2 (Figure 1). The PCR fragment vector2 was cloned in pCR4Blunt-TOPO yielding pUD267. The plasmids were checked by restriction analysis to verify the orientation of the cloned fragment.

### Gene deletion cassette

For the construction of the final gene deletion fragments, 500 base pair sequences from the 5' and 3' ends of *ScARO10,* a gene that encodes a phenyl pyruvate decarboxylase involved in the Ehrlich pathway [Vuralhan. et al., (2005). Appl Environ Microbiol 71 3276-3284] were amplified from CBS1483 genomic DNA.

**Table V Standard vector construction plasmids**

| ***Plasmid*** | ***Fragment*** | ***Template*** | ***Forward Primer*** | ***Reverse Primer*** |
|---|---|---|---|---|
| pUD266 (Topo Blunt + V1) | SceI-pamdS | pUGamdS | Fw V1 NdeI | Rv V1 SceI SacI |
| pUD267 (Topo Blunt + V2) | amdSt-ISceI-SceI | pUD073 | Fw V2 NdeI | Rv V2 SceI EcoRI |
| pUD268 | pUD266 + 5'ScARO10 | CBS1483 | Fw 5'ScARO10 NotI | Rv 5'ScARO10 SacI |
| pUD269 | pUD267 + 3'ScARO10 | CBS1483 | Fw 3'ScARO10 BamHI 80bp | Rv 3'ScARO10 PstI |

In the pUD268 and pUD269 plasmids, the two complete fragments for gene deletion were located between the M13 forward and reverse primers, which were used to amplify the deletion cassette amdS-ISceI.

In the transformation of the different strains, the molar equivalents for two fragments were applied in the yeast transformation mix with the total amount of 837 ng of DNA: 337 ng for vector 1 with the 5' ScARO10 and 500 ng for vector 2 with 3' ScARO10. For *S. pastorianus* CBS1483, the transformation was repeated with 3,37 µg and 5 µg for both pieces, respectively.

### Results

### In vivo assembly recombination of pUDC114

Although there is a lot of data that can be found about transformation efficiencies in CEN.PK113-7D, there is no comparative data for homologous recombination with 60 base pairs available for the strains used in this research. Because polyploidy and chromosomal rearrangements may affect the cells ability to perform homologous recombination, three *S. cerevisiae* species with different ploidy were transformed with the overlapping fragments listed in Table II to generate the pUDC114 plasmid: the haploid CEN.PK113-7D generating IMC067, the diploid CEN.PK122 generating IMC076 and the polyploid PRG410 generating IMC077. Furthermore, the polyploid *S. pastorianus* strains CMBS33 and CBS1483 and the recently discovered diploid *S. eubayanus* CBS12357 [Libkind et al., (2011). PNAS USA 108: 14539-14544] were studied for the ability to produce the pUDC114 plasmid, generating IMC064 for CBS12357 and IMC066 for CMBS33. The expectation for CBS1483 was that the assembly does not happen due to the short length of the homology sequences.

The strains IMC067, IMC076, IMC077, IMC066 and IMC064 were able to recombine the pUDC114 plasmid to produce the amdS gene and grow on acetamide as sole nitrogen source. As expected, the CBS1483 was not able to recombine the 60 base pair overlapping sequences that forms the pUDC114 plasmid. Transformants per µg DNA were calculated. Results are shown in table VI.

**Table VI: Average transformation efficiencies measured in transformants per µg DNA with standard error of the mean (SEM). The transformation of PRG410 for generating IMC077 was performed several times, but only one plate contained positive orange colonies***

| Strain | Transformants per µg DNA | Standard Error of the Mean |
|---|---|---|
| IMC067 (CEN.PK113-7D) | 20563 | 1515 |
| IMC076 (CEN.PK122) | 5952 | 433 |
| IMC077 (PRG410)* | 19 | - |
| IMC064 (CBS 12357) | 364 | 53 |
| IMC066 (CMBS33) | 278 | 85 |
| CBS1483 | 0 | - |

More than 2*10⁴ transformants per µg DNA were formed in the haploid IMC067, approximately 6*10³ transformants per µg DNA were produced in the diploid IMC076 and in the IMC077 only 19 transformants per µg DNA were created from the polyploid ale strain PRG410. Thus, with increasing ploidy of the different *S. cerevisiae* strains, the amount of transformants per µg DNA decreases significantly according to the data presented in Table VI. In the diploid wild type *S. eubayanus* CBS 12357, the amount of transformants per µg DNA was also more than 10 times lower than in the diploid laboratory strain CEN.PK122 (Table VI). The lager brewing strain *S. pastorianus* CMBS33 was not very efficient compared to the diploid *S. cerevisiae* laboratory strain and only slightly less efficient than the other parental strain *S. eubayanus* CBS 12357.

### Construction of the standard vectors

To generate the bi-partite selection marker, the amdS marker was split in two part that shared an overlap of 400 base pairs within the amdS ORF. The first part was obtained by amplifying the Ashbya gossipii *TEF2* promoter and the first 1138 nucleotides of the amdS open reading frame from pUGamdSY. The resulting fragment of 1569 base pairs harbored on its 5' a SceI endonuclease restriction site. The cloning in pCR4Blunt-TOPO plasmid of this fragment resulted in pUD266. The second part of the bi partite marker system was constructed in two steps: 1) the second part of the marker included the last 908 base pairs of the amdS selectable marker and the AgTEF2 terminator. This cassette was flanked on its 3' end by an extension of 60 bp complementary to the *SCEI* cassette. 2) The endonuclease SCEI cassette which carried the *SCEI* gene under the control of the *GAL1* promoter was amplified from pUDC073. On its 3' end this fragment harbored an additional endonuclease SceI restriction site. Subsequently, the two fragments were connected together by fusion PCR and the resulting fused fragment was cloned into the pCR4Blunt-TOPO vector yielding pUDC267.

To control the direction of integration, restriction analysis was performed on pUDC266 with double digestion with NotI and BamH1, with Not1 cutting inside pCR4Blunt-TOPO plasmid and BamHI cutting only inside vector 1, which resulted in a characteristic band pattern of 438 and 5087 base pairs. For direction of integration control in pUD267, the restriction analysis was carried out with NotI and HindIII digestion resulting in a characteristic band pattern of 815 and 5732 base pairs.

The bi-partite fragment contained in pUDC266 was sequenced and revealed no mutation.

Restriction analyses and sequence analyses confirmed the correctness of the plasmids and that no mutation happened in the designed sequence. However, analysis of pUDC267 and the acquired consensus sequence showed several single nucleotide polymorphisms (SNP's). One SNP actually gave a change in the first nucleotide of a codon from amdS, changing the amino acid from tryptophan into arginine. Even though this meant a change from a hydrophobic amino acid into a positively charged (thus hydrophilic) amino acid, no change in effectiveness of the acetamidase growth was discovered. The other SNP's and even the addition of 7 nucleotides were outside the sequences of both amdS and I-SceI.

### Acquiring the deletion cassette

Although the central part of the gene deletion cassette amdS-ISceI was ligated in the standard vectors pUD266 and pUD267, the homologous recombination sequences were not integrated on the deletion cassette. For producing the homologous recombination fragments, genomic DNA of CBS1483 was amplified with Fw 5'ScARO10 NotI and Rv 5'ScARO10 SacI for the upstream part of the ScARO10 and with Fw 3'ScARO10 BamHI 80bp and Rv 3'ScARO10 PstI for the downstream part. The pUDC266 and pUD267 were digested with NotI/SacI and PstI/PmeI and ligated with the 5' fragment (NotI/SacI) and 3' fragment (PstI prepared), respectively. These ligations generated two new vectors pUD268 carrying the first selectable cassette element targeting the 5' side of the *ScARO10* locus and pUD269 carrying the second selectable cassette element targeting the 3' side of the *ScARO10* locus.

### Deletion of ScARO10 allele.

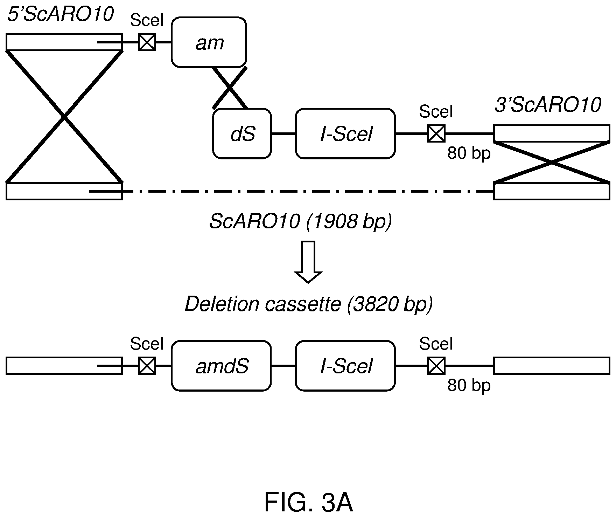

For the proof of principle of gene deletion and marker recovery using this new method of an amdS-ISceI cassette with 500 base pairs homologous flanks, the first step is to successfully delete genes in several strains. The targeted gene deletion with the two previously generated cassettes consisted of three essential recombination steps based on homology (Figure 3A).

The non-functional amdS parts located within both standard vectors were required to recombine and form a functional selectable amdS gene for growth on acetamide. The other two steps were recombination between the *5' ScARO10* and *3'ScARO10* fragments on the cassette and those sites in the genomic DNA.

To evaluate whether this new method for gene deletion and recovery of the marker could be applied to *S. pastorianus,* several strains were tested. As control, three *Saccharomyces cerevisiae* strains were transformed, the laboratory haploid MATa strain CEN.PK113-7D, The laboratory diploid MATa/MATa strain CEN.PK122 and the industrial ale strain PRG410. Along these yeast stains two *Saccharomyces pastorianus* strains CBS1483 and CMBS33 were also transformed.

Deletion of the *ScARO10* allele in strains of the CEN.PK family using the bi-partite approach yielded more than 200 transformants per □g DNA. In contrast transformation of the industrial strain PRG410 yielded a much lower number of transformants (0.4 transformant per □g DNA).

While deletion with short flanks PCR generated deletion cassette was unsuccessful in *S. pastorianus* CBS1483, the application of the bi-partite approach enabled the successful identification of correctly deleted mutants. However, the number of transformants remained low (0.5 transformant per □g DNA) (Table VII). A representative transformation of CBS1483 with 8.37 µg of the bi-partite amdS marker targeting the *ScARO10* locus led to growth of 2 to 9 transformants on 30 mM acetamide medium plate.

From a transformation resulting in nine CBS1483 transformants, two positive colonies were re-streaked to obtain true single colonies and the other 7 colonies were checked via high-fidelity colony PCR with outside-inside primers Fw 5'ScARO10/amdS check and KanB r, generating a fragment of 2851 base pairs. All of them were positive for the homologous recombination between the 5' ScARO10 part and the 400 base pairs overlap within amdS, suggesting the deletion cassettes were all integrated successfully. A single colony isolate was obtained and renamed IMK490 (CBS1483 with one deleted locus *Scaro10Δ::amdS*).

Similarly single colony isolate was obtained from CEN.PK113-7D, CEN.PK122, PRG410, CMBS33 and renamed IMK386, IMK487, IMK488 and IMK489 respectively.

**Table VII Average transformation efficiencies measured in transformants per µg DNA with standard error of the mean (SEM)**

| **Strain** | **host** | **Transformants per µg DNA** | **SEM** |
|---|---|---|---|
| IMK486 | CEN.PK113-7D | 215.9 | 27.4 |
| IMK487 | CEN.PK122 | 212.7 | 25.9 |
| IMK488 | PRG410 | 0.4 | 0.2 |
| IMK489 | CMBS33 | 27.9 | 7.2 |
| IMK490 | CBS1483 | 0.5 | 0.4 |

To be absolutely sure that the deletion cassette was successfully integrated into the targeted genomic DNA, the homologous recombination of the overlap within amdS was also controlled with amdS primers KanA f and KanB r (2164 bp) and the homologous recombination of the 3' ScARO10 with inside-outside primers FK072 and Rv 3'ScARO10/amdS check (1081 bp). Because each strain contains more than one copy of ScARO10, except for CEN.PK113-7D, the presence of other copies of the ScARO10 was controlled with the primers ScARO10-Fw inside and ScARO10-Rv inside (959 bp).

The results showed that all strains used in this project were capable of knocking out at least one copy of ScARO10. In CEN.PK113-7D scaro10Δ::amdS, the other strains, including both colonies checked for IMK490, still had at least one copy of the ScARO10 in their genome. The IMK486-IMK490 colonies were all stocked and a sample of each one was re-inoculated in YPD to grow for the second step, the marker removal.

### Seamless marker removal

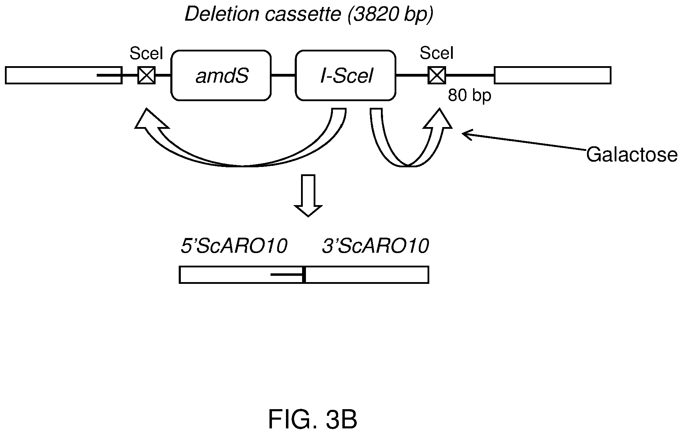

To verify that the integrated bi-partite construct could be easily recovered from the targeted locus using the inducible endonuclease *SCEI,* the strain IMK490 (CBS1483 with one deleted locus *Scaro10Δ::amdS*) was grown on a galactose medium to induce the *GAL1* promoter that is controlling the expression of *SCEI.* (Figure 3B). The strain IMK490 was grown on synthetic medium with galactose for 48hours. Upon induction, the endonuclease creates a cut at the SceI sites that flank the deletion cassette and in the meantime removes its coding region from the chromosome, therefore enabling a recycling of the genome editing construct.

The strain IMK386, IMK487, IMK488 and IMK489 were treated similarly.

The selection for strains with counter selected marker was performed using two different methods:

### 1- Plating on galactose

The galactose grown IMK490 cells were streaked on plates containing galactose as carbon source and were incubated at 30°C for 3 days. Single colony isolates were resuspended in 100 □l of sterile water and 5 □l were transferred on synthetic media plates containing either ammonium or acetamide as sole nitrogen source. These plates were grown for 2 days at 30°C. Similarly single isolate colonies of IMK386, IMK487, IMK488 and IMK489 were spotted on synthetic media plates containing either ammonium or acetamide as sole nitrogen source.

For IMK486, out of the 5 picked colonies none had recycled the amdS marker, for IMK487 only 1 out of the 5 clones had recycled the amdS marker, and for IMK489 all five picked clones had lost the amdS marker. For the strain IMK490 11 colonies were checked and 10 had excised the amdS selectable marker. To determine whether the marker was removed from the chromosome a colony PCR was performed on the genomic DNA of a representative clone with the outside-outside primers Fw 5'ScARO10/amdS check and Rv 3'ScARO10/amdS check, which produced a fragment of 1426 base pairs. This confirmed that the cassette amdS-ISceI had been removed from the genome.

### 2-Inoculation in liquid medium and counter selection on fluoroacetamide.

The second method used to remove the marker, consisted in inoculating 1 mL of a culture from each strain containing the amdS-ISceI cassette in liquid medium with 2% galactose as main carbon source and 0.05% glucose to enhance growth of the different yeast strains. After growth for 4 hours in this liquid medium, samples were taken and diluted 200 times in sterile water and 100□l were platted on synthetic medium with galactose and fluoroacetamide plates. Colonies that express the amdS gene would therefore hydrolyze fluoroacetamide in ammonium and fluoroacetate, which is toxic. Only cells having lost the amdS selectable marker would grow.

In this project, homologous recombination was investigated by *in vivo* assembly of the plasmid pUDC114 from four double stranded DNA fragments that had 60 base pair overlaps. pUDC114 contained the carotene genes crtYBEI, which gave the positive colonies an orange colour as a means for quick identification. The transformation results showed that with increasing ploidy of the different S. cerevisiae strains, the amount of transformants per µg DNA decreased significantly, from 2*10⁴ transformants per µg DNA in haploid CEN.PK113-7D, to 6*10³ transformants per µg DNA in the diploid CEN.PK122 and finally to 19 transformants per µg DNA in the polyploid ale brewing strain PRG410. In the recently discovered diploid wild type *S. eubayanus* CBS 12357, the amount of transformants per µg DNA was also more than 10 times lower than in the diploid laboratory strain CEN.PK122.

The efficiency of homologous recombination in the complex lager brewing strains was not investigated before. The *S. pastorianus* CMBS33 was 20 times less efficient than the diploid CEN.PK122, but just slightly less efficient than the other parental strain *S. eubayanus* CBS12357. The transformation efficiency and homologous recombination of the pUDC114 fragments in lager brewing strain CMBS33 was ±300 transformants per µg DNA, but zero for CBS1483. This proves that not all lager brewing strains are identical, with genetic differences determined by the particularities of the brewing process they were selected from.

The gene deletion system with the amdS marker removal by the endonuclease I-SceI is a novel way to alter and delete genes in lager brewing strains and laboratory *S. cerevisiae* strains. The possibility of altering and/or deleting genes and subsequent marker removal in *S. pastorianus* CBS1483 with the amdS-ISceI cassette contributes substantially to the toolbox of researchers in the brewing industry.

### Example 2

Genomic DNA of the *Kluyveromyces lactis* strain ATCC 8585 was prepared as described (Burke et al., 2000. Cold Spring Harbor Laboratory. Methods in yeast genetics: a Cold Spring Harbor Laboratory course manual). ORF KLLA0F27995g, encoding KlGBU1, was amplified from genomic DNA using Phusion Hot-Start polymerase (Finnzymes) and primers GBU1 forward primer (5'-CATCCGAACATAAACAACCATGAA GGTTGCAGGATTTATATTG) and GBU1 reverse primer (5'-CAAGAAT CTTTTTATTGTCAGTACTGATCAGGCTTGCAAAACAAATTGTTC). The coding sequence of the *K. lactis* GBU1 gene was obtained.

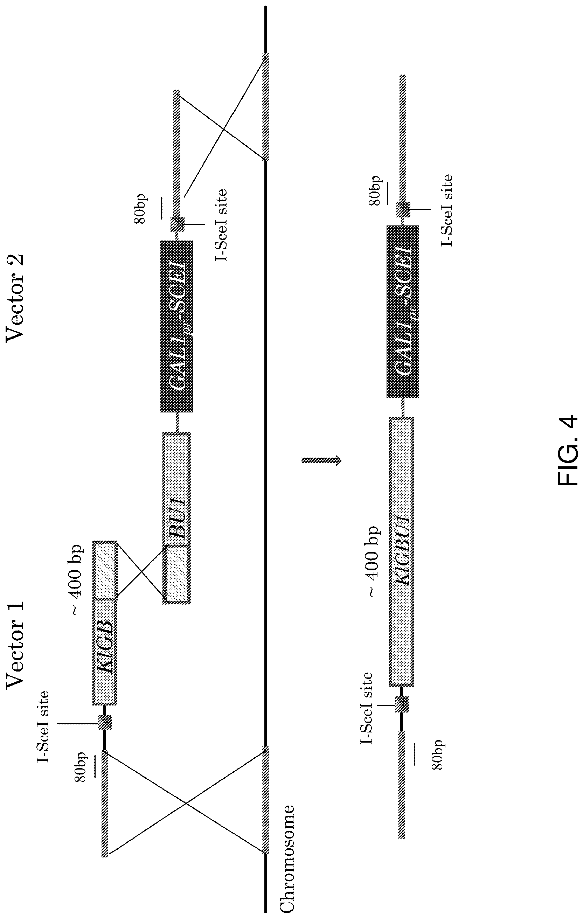

A set of targeting constructs comprising the selection marker *KlGBU1* with all essential parts for the standard deletion cassette is provided in Figure 4. The 400 base overlap in the selection marker *KlGBU1* (indicated by a cross) is designed to recombine due to the homology.

## Claims

1. A set of targeting constructs, comprising a first construct comprising, in this order, a first region of homology with a target genome of a microorganism, a recognition site for an endonuclease, and a first part of a selection marker, and a second construct comprising, in this order, a second part of the selection marker, a copy of the endonuclease recognition site, and a second region of homology with the target genome of the microorganism, whereby
the first and second regions of homology with the target genome each comprises at least 20 base pairs (bp);
a fragment of the first part of the selection marker overlaps with a fragment that is present in the second part of the selection marker, allowing recombination between the first and second part of the selection marker, said fragment preferably comprising between 50 and 600 bp;
a coding sequence that encodes the endonuclease and which is coupled to an inducible promoter is present on the first or second construct; and
a part of the first region of homology with the target genome on the first construct is duplicated between the copy of the endonuclease recognition site and the second region of homology with the target genome on the second construct; or a part of the second region of homology with the target genome on the second construct is duplicated between first region of homology with the target genome and the endonuclease recognition site on the first construct, said duplicated region comprising between 20 and 200 bp.

2. The set of targeting constructs according to claim 1, wherein the overlapping fragment of the selection marker is about 200 base pairs (bp).

3. The set of targeting constructs according to claim 1 or claim 2, wherein the duplicated region of homology with the target genome on the first and second targeting construct preferably is about 80 bp.

4. The set of targeting constructs according to any one of the previous claims, whereby the endonuclease is a homing endonuclease.

5. The set of targeting constructs according to any one of the previous claims, whereby the selectable marker is an auxothrophic marker and/or a dominant marker.

6. The set of targeting constructs according to any one of the previous claims, whereby the inducible promoter is selected from GAL1 promoter, GAL10 promoter, SUC2 promoter, MAL12 promoter, CUP1, and a tetracycline-regulatable promoter.

7. The set of targeting constructs according to any one of the previous claims, wherein the microorganism is an aneuploid microorganism.

8. The set of targeting constructs according to any one of the previous claims, wherein the microorganism is an Ascomycota, preferably a Saccharomycotina.

9. The set of targeting constructs according to any one of the previous claims, wherein the microorganism is Saccharomyces pastorianus.

10. A method for altering a genome in a microorganism, comprising providing the set of targeting constructs according to any one of claims 1-9 to said microorganism, and selecting a microorganism in which the genome has been altered.

11. A method for producing a microorganism comprising a genomic alteration, the method comprising providing the set of targeting constructs according to any one of claims 1-9 to said microorganism, and selecting a microorganism in which the genome has been altered and that functionally expresses the recombined selection marker.

12. The method according to claim 11, further comprising inducing the inducible promoter for expression of the endonuclease.

13. A microorganism, comprising an genomic alteration that is produced by the method of claim 11.

14. A microorganism, comprising a genomic alteration, the alteration comprising insertion of a functional, recombined selection marker and a coding sequence that encodes a endonuclease and that is coupled to an inducible promoter, whereby the insertion site comprises one copy of a recognition sequence for the endonuclease on both sites of the insertion.

15. A method for producing a microorganism comprising an altered chromosomal region, the method comprising providing the microorganism according to claim 14, and inducing the inducible promoter to remove the nucleic acid sequences in between the recognition sequences of the endonuclease.

## Patentansprüche

1. Satz von Targeting-Konstrukten, umfassend ein erstes Konstrukt, umfassend, in dieser Reihenfolge, eine erste Region mit Homologie mit einem Zielgenom eines Mikroorganismus, eine Erkennungsstelle für eine Endonuklease und einen ersten Teil eines Selektionsmarkers, und ein zweites Konstrukt, umfassend, in dieser Reihenfolge, einen zweiten Teil des Selektionsmarkers, eine Kopie der Endonuklease-Erkennungsstelle und eine zweite Region mit Homologie mit dem Zielgenom des Mikroorganismus, wobei
die ersten und zweiten Regionen mit Homologie mit dem Zielgenom jeweils mindestens 20 Basispaare (bp) umfassen;
ein Fragment des ersten Teils des Selektionsmarkers ein Fragment überlappt, das in dem zweiten Teil des Selektionsmarkers vorhanden ist, was die Rekombination zwischen dem ersten und zweiten Teil des Selektionsmarkers erlaubt, das Fragment bevorzugt umfassend zwischen 50 und 600 bp;
eine Codierungssequenz, die die Endonuklease codiert und die an einen induzierbaren Promoter gekoppelt ist, auf dem ersten oder zweiten Konstrukt vorhanden ist;
ein Teil der ersten Region mit Homologie mit dem Zielgenom auf dem ersten Konstrukt zwischen der Kopie der Endonuklease-Erkennungsstelle und der zweiten Region mit Homologie mit dem Zielgenom auf dem zweiten Konstrukt dupliziert ist; oder ein Teil der zweiten Region mit Homologie mit dem Zielgenom auf dem zweiten Konstrukt zwischen der ersten Region mit Homologie mit dem Zielgenom und der Endonuklease-Erkennungsstelle auf dem ersten Konstrukt dupliziert ist, welche duplizierte Region zwischen 20 und 200 bp umfasst.

2. Satz von Targeting-Konstrukten nach Anspruch 1, wobei das überlappende Fragment des Selektionsmarkers ungefähr 200 Basispaare (bp) ist.

3. Satz von Targeting-Konstrukten nach Anspruch 1 oder Anspruch 2, wobei die duplizierte Region mit Homologie mit dem Zielgenom auf dem ersten und zweiten Targeting-Konstrukt bevorzugt ungefähr 80 bp ist.

4. Satz von Targeting-Konstrukten nach einem der vorhergehenden Ansprüche, wobei die Endonuklease eine Homing-Endonuklease ist.

5. Satz von Targeting-Konstrukten nach einem der vorhergehenden Ansprüche, wobei der selektierbare Marker ein auxothroper Marker und/oder ein dominanter Marker ist.

6. Satz von Targeting-Konstrukten nach einem der vorhergehenden Ansprüche, wobei der induzierbare Promoter ausgewählt ist aus GAL1-Promoter, GAL10-Promoter, SUC2-Promoter, MAL12-Promoter, CUP1 und einem Tetracyclin-regulierbaren Promoter.

7. Satz von Targeting-Konstrukten nach einem der vorhergehenden Ansprüche, wobei der Mikroorganismus ein aneuploider Mikroorganismus ist.

8. Satz von Targeting-Konstrukten nach einem der vorhergehenden Ansprüche, wobei der Mikroorganismus ein Ascomycota, bevorzugt ein Saccharomycotina, ist.

9. Satz von Targeting-Konstrukten nach einem der vorhergehenden Ansprüche, wobei der Mikroorganismus Saccharomyces pastorianus ist.

10. Verfahren zur Veränderung eines Genoms in einem Mikroorganismus, umfassend das Bereitstellen des Satzes von Targeting-Konstrukten nach einem der Ansprüche 1-9 für den Mikroorganismus und Auswählen eines Mikroorganismus, in dem das Genom verändert wurde.

11. Verfahren zur Herstellung eines Mikroorganismus, umfassend eine genomische Veränderung, das Verfahren umfassend das Bereitstellen des Satzes von Targeting-Konstrukten nach einem der Ansprüche 1-9 für den Mikroorganismus und Auswählen eines Mikroorganismus, in dem das Genom verändert wurde und der funktional den rekombinierten Selektionsmarker exprimiert.

12. Verfahren nach Anspruch 11, ferner umfassend das Induzieren des induzierbaren Promoters, um die Endonuklease zu exprimieren.

13. Mikroorganismus, umfassend eine genomische Veränderung, die durch das Verfahren nach Anspruch 11 erzeugt wird.

14. Mikroorganismus, umfassend eine genomische Veränderung, die Veränderung umfassend Insertion eines funktionalen, rekombinierten Selektionsmarkers und einer Codierungssequenz, die eine Endonuklease codiert und die an einen induzierbaren Promoter gekoppelt ist, wobei die Einsetzstelle eine Kopie einer Insertionssequenz für die Endonuklease an beiden Stellen der Insertion umfasst.

15. Verfahren zur Herstellung eines Mikroorganismus, umfassend eine veränderte chromosomale Region, das Verfahren umfassend das Bereitstellen des Mikroorganismus nach Anspruch 14 und Induzieren des induzierbaren Promoters, um die Nukleinsäuresequenzen zwischen den Erkennungssequenzen der Endonuklease zu entfernen.

## Revendications

1. Ensemble de constructions de ciblage, comprenant une première construction comprenant, dans cet ordre, une première région d'homologie avec un génome cible d'un micro-organisme, un site de reconnaissance pour une endonucléase, et une première partie d'un marqueur de sélection, et une seconde construction comprenant, dans cet ordre, une seconde partie du marqueur de sélection, une copie du site de reconnaissance d'endonucléase, et une seconde région d'homologie avec le génome cible du micro-organisme, selon lequel
les première et seconde régions d'homologie avec le génome cible comprennent chacune au moins 20 paires de bases (pb) ;
un fragment de la première partie du marqueur de sélection se chevauche avec un fragment qui est présent dans la seconde partie du marqueur de sélection, permettant une recombinaison entre les première et seconde parties du marqueur de sélection, ledit fragment comprenant de préférence entre 50 et 600 pb ;
une séquence codante qui code pour l'endonucléase et qui est couplée à un promoteur inductible présent sur la première ou la seconde construction ; et
une partie de la première région d'homologie avec le génome cible sur la première construction est dupliquée entre la copie du site de reconnaissance d'endonucléase et la seconde région d'homologie avec le génome cible sur la seconde construction ; ou une partie de la seconde région d'homologie avec le génome cible sur la seconde construction est dupliquée entre la première région d'homologie avec le génome cible et le site de reconnaissance d'endonucléase sur la première construction, ladite région dupliquée comprenant entre 20 et 200 pb.

2. Ensemble de constructions de ciblage selon la revendication 1, dans lequel le fragment chevauchant du marqueur de sélection est d'environ 200 paires de bases (pb) .

3. Ensemble de constructions de ciblage selon la revendication 1 ou la revendication 2, dans lequel la région dupliquée d'homologie avec le génome cible sur la première et la seconde construction de ciblage est de préférence d'environ 80 pb.

4. Ensemble de constructions de ciblage selon l'une quelconque des revendications précédentes, selon lequel l'endonucléase est une endonucléase de ciblage.

5. Ensemble de constructions de ciblage selon l'une quelconque des revendications précédentes, selon lequel le marqueur sélectionnable est un marqueur auxotrophe et/ou un marqueur dominant.

6. Ensemble de constructions de ciblage selon l'une quelconque des revendications précédentes, selon lequel le promoteur inductible est choisi parmi le promoteur GAL1, le promoteur GAL10, le promoteur SUC2, le promoteur MAL12, CUP1, et un promoteur régulable par la tétracycline.

7. Ensemble de constructions de ciblage selon l'une quelconque des revendications précédentes, dans lequel le micro-organisme est un micro-organisme aneuploïde.

8. Ensemble de constructions de ciblage selon l'une quelconque des revendications précédentes, dans lequel le micro-organisme est un *Ascomycota,* de préférence un *Saccharomycotina.*

9. Ensemble de constructions de ciblage selon l'une quelconque des revendications précédentes, dans lequel le micro-organisme est *Saccharomyces pastorianus.*

10. Méthode pour la modification d'un génome dans un micro-organisme, comprenant la fourniture de l'ensemble de constructions de ciblage selon l'une quelconque des revendications 1 à 9 au dit micro-organisme, et la sélection d'un micro-organisme dans lequel le génome a été modifié.

11. Méthode pour la production d'un micro-organisme comprenant une modification génomique, la méthode comprenant la fourniture de l'ensemble de constructions de ciblage selon l'une quelconque des revendications 1 à 9 au dit micro-organisme, et la sélection d'un micro-organisme dans lequel le génome a été modifié et qui exprime fonctionnellement le marqueur de sélection recombiné.

12. Méthode selon la revendication 11, comprenant en outre l'induction du promoteur inductible pour l'expression de l'endonucléase.

13. Micro-organisme, comprenant une modification génomique qui est produit par la méthode selon la revendication 11.

14. Micro-organisme, comprenant une modification génomique, la modification comprenant l'insertion d'un marqueur de sélection recombiné fonctionnel et d'une séquence codante qui code une endonucléase et qui est couplée à un promoteur inductible, selon lequel le site d'insertion comprend une copie d'une séquence de reconnaissance pour l'endonucléase sur les deux sites de l'insertion.

15. Méthode pour la production d'un micro-organisme comprenant une région chromosomique modifiée, la méthode comprenant la fourniture du micro-organisme selon la revendication 14, et l'induction du promoteur inductible pour éliminer les séquences d'acide nucléique situées entre les séquences de reconnaissance de l'endonucléase.
